# EUROPEAN PATENT APPLICATION

(11) **EP 2 101 176 A1**
(43) Date of publication of application: **16.09.2009**
(21) Application number: 08102363.2
(22) Date of filing: 06.03.2008
(51) Int. Cl.: G01N 33/569, C07K 14/38

(54) **Diagnosis of and vaccination against Aspergillus fumigatus**

(71) Applicant: Technische Universität Braunschweig, 38106 Braunschweig (DE)
(72) Inventor: Schütte, Mark, 38114 Braunschweig (DE); Hust, Michael, 30169 Hannover (DE); Dübel, Stefan, 38108 Braunschweig (DE)
(74) Representative: Taruttis, Stefan Georg

(57) **Abstract**

The present invention is based on the finding that a protein, presently termed Crf2 is an antigen that is specific for an invasive *Aspergillus* infection in animals, at least in humans and in birds, which elicits an immune response specifically directed against *Aspergillus fumigatus*.

In accordance with the specificity of Crf2 for an invasive *Aspergillus* infection, Crf2 and antibody elicited by Crf2 in an infected animal can be used in a process for diagnosing an invasive *Aspergillus* infection without detecting a *Candida* infection.

SEQUENCE LISTING

<110>  Technische Universität Braunschweig

<120>  Diagnosis of and vaccination against Aspergillus fumigatus

<130>  T1005EP

<160>  1

<170>  PatentIn version 3.4

<210>  1
<211>  333
<212>  PRT
<213>  Aspergillus fumigatus

<400>  1

Met Tyr Phe Lys Tyr Thr Ala Ala Ala Leu Ala Ala Val Leu Pro Leu
1               5                   10                  15

Cys Ser Ala Gln Thr Trp Ser Lys Cys Asn Pro Leu Glu Lys Thr Cys
            20                  25                  30

Pro Pro Asn Lys Gly Leu Ala Ala Ser Thr Tyr Thr Ala Asp Phe Thr
        35                  40                  45

Ser Ala Ser Ala Leu Asp Gln Trp Glu Val Thr Ala Gly Lys Val Pro
    50                  55                  60

Val Gly Pro Gln Gly Ala Glu Phe Thr Val Ala Lys Gln Gly Asp Ala
65                  70                  75                  80

Pro Thr Ile Asp Thr Asp Phe Tyr Phe Phe Phe Gly Lys Ala Glu Val
                85                  90                  95

Val Met Lys Ala Ala Pro Gly Thr Gly Val Val Ser Ser Ile Val Leu
            100                 105                 110

Glu Ser Asp Asp Leu Asp Glu Val Asp Trp Glu Val Leu Gly Gly Asp
        115                 120                 125

Thr Thr Gln Val Gln Thr Asn Tyr Phe Gly Lys Gly Asp Thr Thr Thr
    130                 135                 140

Tyr Asp Arg Gly Thr Tyr Val Pro Val Ala Thr Pro Gln Glu Thr Phe
145                 150                 155                 160

His Thr Tyr Thr Ile Asp Trp Thr Lys Asp Ala Val Thr Trp Ser Ile
                165                 170                 175

Asp Gly Ala Val Val Arg Thr Leu Thr Tyr Asn Asp Ala Lys Gly Gly
            180                 185                 190

Thr Arg Phe Pro Gln Thr Pro Met Arg Leu Arg Leu Gly Ser Trp Ala
        195                 200                 205

Gly Gly Asp Pro Ser Asn Pro Lys Gly Thr Ile Glu Trp Ala Gly Gly
    210                 215                 220

Leu Thr Asp Tyr Ser Ala Gly Pro Tyr Thr Met Tyr Val Lys Ser Val
225                 230                 235                 240

Arg Ile Glu Asn Ala Asn Pro Ala Glu Ser Tyr Thr Tyr Ser Asp Asn
                245                 250                 255

Ser Gly Ser Trp Gln Ser Ile Lys Phe Asp Gly Ser Val Asp Ile Ser
            260                 265                 270

Ser Ser Ser Ser Val Thr Ser Ser Thr Thr Ser Thr Ala Ser Ser Ala
        275                 280                 285

Ser Ser Thr Ser Ser Lys Thr Pro Ser Thr Ser Thr Leu Ala Thr Ser
    290                 295                 300

Thr Lys Ala Thr Pro Thr Pro Ser Gly Thr Ser Ser Gly Ser Asn Ser
305                 310                 315                 320

Ser Ser Ser Ala Glu Pro Thr Ala Ala Val Leu Ala Phe
                325                 330

## Description

The present invention relates to an antigen specific for invasive aspergillosis and the use of the antigen for a diagnostic test as well as the use of the antigen as a vaccine. The antigen is highly specific for aspergillosis and therefore the diagnostic process is capable of differentiating an infection by *Aspergillus fumigatus* from an infection by *Candida*, especially *Candida albicans*. The vaccine against aspergillosis is also based on the immunogenic properties of the antigen. Both the diagnostic test for *Aspergillus fumigatus* and the vaccine eliciting an immune response inducing immunoprotection against *Aspergillus fumigatus* are specific for *Aspergillus fumigatus*.

### State of the art

Bozza et al. (Microbes and Infection 4 (2002) 1281-1290) show that the administration of a protein termed Aspf16 obtained from *Aspergillus* when used as a vaccine did not result in an effective immunoprotection against subsequent challenge with viable *Aspergillus* conidia in immunosuppressed mice. In contrast, administration of the immunogenic ODN 1668 (TCC-ATC-ACG-TTC-CTG-ATG-CT) sequence was necessary to obtain an immune response to Aspf16 to protect mice against invasive *Aspergillus* infection. The effect of increasing the immunoprotective properties of proteins by combination with the ODN sequence was also shown for different *Aspergillus* proteins. Prior to the experimental immune challenge with *Aspergillus* conidia, the immune system of the test animals was affected by administration of the general suppressant of cell growth cyclophosphamid.

Denikus et al. (Infection and Immunity (2005) 4704 - 4713) quotes Bozza et al. in respect of the immunoprotective potential of Aspf16 without mentioning that Bozza et al. only found immunoprotection when administering recombinant proteins in combination with the ODN sequence 1668, which is known from Lipford et al. (Eur. J. Immunol. 27 (1997) 3420-3426). Denikus et al. identified an immune response against Aspf16 in *Aspergillus* infected rabbits.

Boywer and Denning (2007) postulate that the sequence for the Aspf16 described by Bozza et al. is no naturally occurring protein but the consequence of a frame-shift or a sequencing error.

During the preparation of the present invention, it has been found that an mRNA sequence encoding a protein of the size of Aspf16 as given in Bozza et al. could not be identified in total mRNA isolated from biopsies of patients with proven invasive aspergillosis.

### Objects of the invention

In view of prior art publications, especially Bozza et al., it is an object of the present invention to provide a novel protein that can be used as an antigen for eliciting an immune response specific for *Aspergillus*, especially *Aspergillus fumigatus*, especially for use in humans having an immune system impaired by depletion of neutrophils.

Further, it is an object of the present invention to provide the use of a protein and of antibodies specific for that protein, respectively, in a test kit for diagnosis and a diagnostic method for detecting a invasive aspergillosis, especially by *Aspergillus fumigatus*, without interaction with *Candida* or antibody directed against Candida, especially *Candida albicans*.

### General description of the invention

The present invention is based on the finding that a protein, presently termed Crf2 and having the amino acid sequence Seq ID No. 1, is an antigen that is specific for an invasive *Aspergillus* infection in animals, preferably at least in humans and in birds, and which protein elicits an immune response specifically directed against *Aspergillus*, especially against *Aspergillus fumigatus*.

In accordance with the specificity of Crf2 for an invasive *Aspergillus* infection, Crf2 and antibody elicited by Crf2 in an infected animal can be used in a process for diagnosing an invasive *Aspergillus* infection. Accordingly, in a first aspect the present invention is directed to a method for diagnosis by specifically detecting in a biopsy, e.g. in bronchoalveolar lavage fluid (BAL) in serum of an animal, an immune reaction with Crf2. In this diagnostic method, Crf2, preferably obtained by heterologous expression in a micro-organism and purification from the expression host, can be provided, preferably in an immobilized state, e.g. bound to a carrier substrate, and contacted with a sample, e.g. serum obtained from an animal, preferably from a human or a bird, followed by removal of antibody not bound to Crf2 and, subsequently, detection of antibody bound to Crf2, which antibody reaction is a specific indicator for invasive aspergillosis.

It has been found that the detection of anti-Crf2 antibody in a sample obtained from an animal, e.g. from serum, using interaction with Crf2 in a diagnostic assay is highly specific for invasive *Aspergillus* infections, especially by *Aspergillus fumigatus*, without detecting an immune response elicited by *Candida*, especially by *Candida albicans*. Further, the diagnostic methods using Crf2 do not give a specific reaction with sera obtained from individuals being infected with asthmatic bronchopulmonary aspergillosis. Accordingly, Crf2 provides the basis for a specific immunological detection of an immune response in a animal, especially in humans for invasive aspergillosis, which does not give a cross reaction with immune responses elicited by an infection by *Candida albicans* or asthmatic bronchopulmonary aspergillosis, i.e. an immunoassay using Crf2 is highly specific for invasive aspergillosis by *Aspergillus fumigatus* without giving unspecific, i.e. false positive reactions with sera containing an immune response to *Candida albicans* or to bronchopulmonary aspergillosis.

When testing cross-reactivity of Crf2 with immune responses elicited by *Candida* infection and or bronchopulmonary aspergillosis, no reaction of the respective sera with Crf2 was found. Further, neither serum from Crf2-immunized nor from PBS-immunized mice showed any reaction with hyphae or spores from *Candida albicans*. Accordingly, Crf2 can be used in a diagnostic test kit and in a diagnostic method, respectively, for specifically detecting invasive aspergillosis, especially by *Aspergillus fumigatus*, without indicating a Candida infection, nor bronchopulmonary aspergillosis.

In another aspect, the present invention on the basis of the high specificity of Crf2 for invasive aspergillosis, especially caused by *Aspergillus fumigatus*, provides for a vaccine containing Crf2 in the form of the protein or in the form of a nucleic acid sequence encoding Crf2, preferably contained in a eucaryotic expression cassette, for specifically eliciting a protective immune response in animals against invasive aspergillosis, especially against invasive aspergillosis by *Aspergillus fumigatus*. It has been found that the immune protection that is elicited by administration of a vaccine composition comprising a protein having, preferably consisting of the epitopes contained in Seq. ID No. 1, is also effective in humans and birds with depletion of neutrophils, e.g. caused subsequent to administration of the vaccine composition of the invention. The maintenance of an effective immune protection originally elicited by the vaccine composition even under the condition of depletion of neutrophils subsequent to administration of the vaccine composition is surprising in view of Bozza et al., because the use of the general cell growth inhibitor cyclophosphamid essentially leaves existing neutrophils intact, which form an effective part in the immune response against invasive aspergillosis.

Accordingly, the present invention provides for the use of Crf2 for the production of a vaccine composition for providing immune protection against *Aspergillus*, especially *Aspergillus fumigatus* in a animal, preferably humans or birds, especially when the recipients subsequent to administration of the vaccine composition have an immune system impaired by depletion of neutrophils.

Administration of a vaccine containing Crf2 according to Seq ID No. 1 or containing a eucaryotic expression cassette, e.g. mammalian, human or for birds, the expression cassette containing a nucleic acid sequence encoding Crf2 of Seq ID No. 1 functionally arranged adjacent a promoter sequence in 5' and a terminator sequence in 3', administration of the vaccine to an animal, exemplified by mice, resulted in immunoprotection of at least 90, preferably at least 95, more preferably 100% of immunized animals. The immune response caused by immunization with Crf2 was demonstrated to provide an efficient immunoprotection against subsequent inoculation with viable *Aspergillus fumigatus* conidia, even when prior to the artificial *Aspergillus* infection the immune system was impaired by depletion of neutrophils. Accordingly, the present invention provides for the use of Crf2 protein having Seq ID No. 1 for the production of a vaccine composition for eliciting a protective immune response in an animal even if the animal subsequent to administration of Crf2 is depleted of neutrophils. The depletion of neutrophils, which are a predominant portion of the natural immune response against invasive aspergillosis, can be caused by immunosuppression due to medical treatment or due to disease.

In contrast to vaccines containing a peptide having the amino acid sequence of Aspf16 as described in Bozza et al., it could be shown that a vaccine consisting of only Crf2 according to Seq ID No. 1 as the specific immunogen, upon immunization produced an efficient immune response against challenge of animals by a viable conidia of *Aspergillus fumigatus*. For protective immunization, Crf2 protein, preferably in combination with formulation agents and/or an adjuvant was used. Preferably, the pharmaceutical composition used as a vaccine did not contain ODN oligonucleotides as described in Bozza et al.

Comparison of detailed analyses of immunized animals in respect of the immune response elicited by a vaccine based on Crf2 according to Seq ID No. 1 as the immunogen compared to the immune response to a vaccine containing Aspf16 as the immunogen showed that the vaccine according to the invention produced a higher titer of antibody specific against *Aspergillus fumigatus* hyphae which are specific for invasive aspergillosis, as well as a higher titer of T-cells (e.g. Th1, Th2) directed against *Aspergillus fumigatus*, including hyphae, especially against *Aspergillus fumigatus* grown under conditions specific for invasive aspergillosis.

### Detailed description of the invention

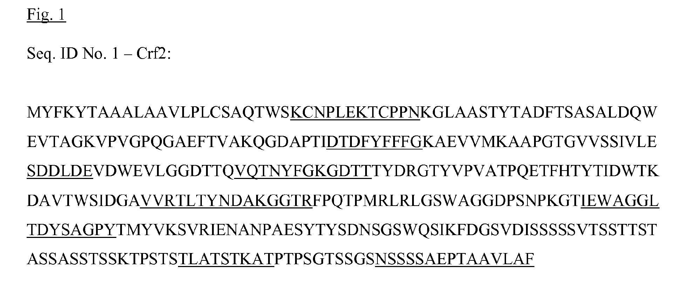

The invention is now described in greater detail by examples with reference to the figures, wherein
- Figure 1 at Seq. ID No 1 gives the amino acid sequence of Crf2,

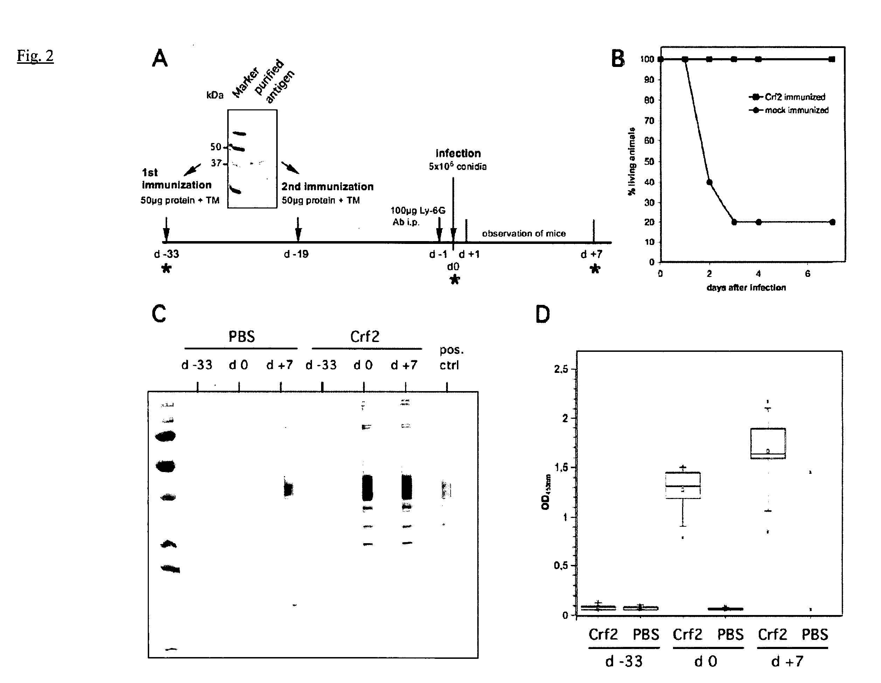

- Figure 2 shows the course of the immunization by Crf2, namely under A) a schematic overview of the course of immunization, immunosuppression and challenge with *Aspergillus* conidia, under B) a graphic representation of the number of immunized and immunosuppressed surviving mice, under C) a Western blot containing Crf2 detected with serum of mice taken during the course of the immunization, and under D) a Whisker plot representing ELISA results from sera taken over the course of the immunization and inoculation with *Aspergillus* conidia, and

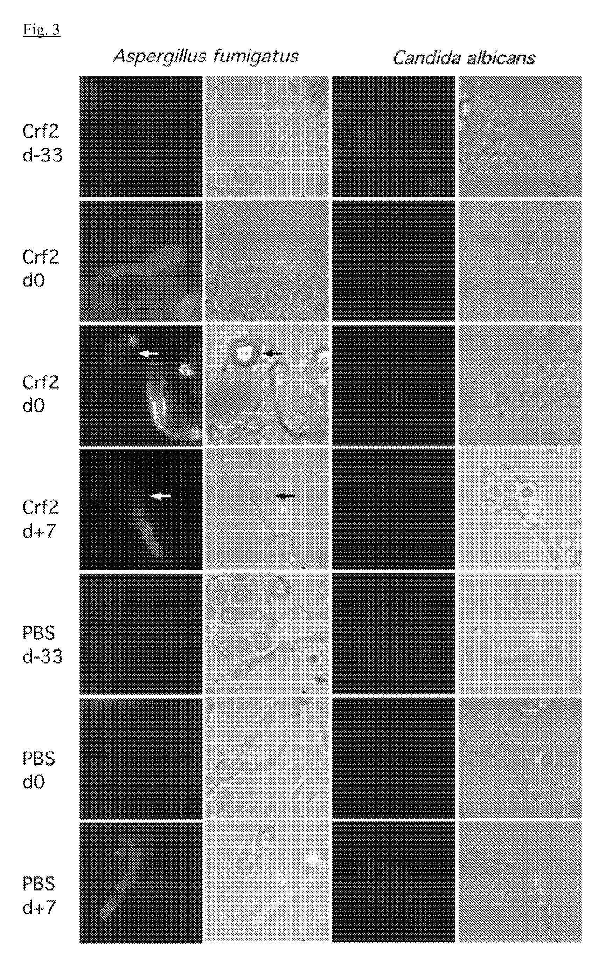

- Figure 3 shows micrographs of *A. fumigatus* and *Candida*, respectively, incubated with sera from Crf2 immunized animals (Crf2) and a mock immunized mouse (PBS) having survived infection with *A. fumigatus* at day 0, in immune fluorescence (left column each) and light microscopy (right column each).

### Example 1: Cloning of the coding sequence for Crf2 and production of Crf2 by heterologous expression

In an attempt to amplify the coding sequence for Aspf16 as described by Bozza et al., primers terminally arranged in the given aspf16 sequence were designed that would be suitable for amplification of the complete coding sequence for full-length Aspf16.

In contrast to Bozza et al., who isolated nucleic acids from an *in vitro* culture of *Aspergillus fumigatus*, the present inventors used cDNA produced by reverse transcription PCR of mRNA isolated from a biopsy obtained from a human patient with proven invasive aspergillosis. In the present invention, no amplificate representing the full-length coding sequence of 1,300 bp of Aspf16 was found when using the primers designed to hybridize to terminal sections of the full length aspf16 sequence. However, two PCR products, one of 855 bp and one of 1008 bp were found, the first encoding Aspf9 and the second not corresponding to a known gene product. However, analysis of the second amplification product identified a coding sequence, presently termed Crf2, encoding a total of 315 amino acids, which essentially correspond to sections of Aspf16 and Crf1, respectively. From the partial similarity of the newly found Crf2 with Crf1, it could be concluded in retrospect that Crf2 is a novel splice variant of Crf1, which splice variant is specific for invasive aspergillosis in humans.

In the reverse transcription reaction, oligo dT primers (Invitrogen, Karlsruhe) were used. First strand cDNA was used as a template for nested PCR, followed by a second amplification using the PCR product of the first PCR reaction. In the sequences given, restriction sites for NheI and NotI are underlined. PCR products were restricted with NheI and NotI and cloned into the expression vector pET 21A (+) (Novagen, Darmstadt), giving an expression vector for Crf2.

For simplification of purification, a second expression vector encoding Crf2 with an added biotin acceptor domain (Cloutier et al., Mol. Immunol. 37, 1067-1077) was cloned by integrating a biotin acceptor domain (BAD) encoding sequence into the XbaI / NheI site of the expression vector, effectively positioning the biotin acceptor domain encoding sequence in 5' to the Crf2 encoding sequence. Alternatively, labelling could be made by attaching an amino acid sequence for strep-tag, flag-tag, E-tag, S-tag, myc-tag or yol-tag to the N-terminus or C-terminus of the immunogenic Crf2 peptide.

In Figure 1, the amino acid sequence of Crf2 according to the present invention is given as Seq. ID No 1, with the underlined sections being epitopes that have been identified by affinity of sera obtained from mice immunized with Crf2 according to Example 3. Due to the dense arrangement of epitopes in Crf2, for the purposes of the invention, the term Crf2 in addition to Seq. ID No 1 also extends to proteins of the same, longer or shorter length than Seq. ID No 1, which contain at least one, preferably 2, 3, 4, 5 or all 6 epitopes identified in Seq. ID No 1, with each epitope present independently in one or more copies.

For expression, XL1 - blue MRF' *E. coli* (Stratagene, Amsterdam) were transformed and grown in standard medium with added glucose and ampicillin and, in the case of the expression vector encoding Crf2 with an added biotin acceptor domain, including 50 µM biotin. After cultivation at 37 °C to an OD₆₀₀ <0.1, IPTG was added to 1 mM and cultivation was continued for 5 hours. Crf2 and Crf2 with an N-terminally added biotin acceptor domain could be isolated from harvested cells after cell disruption, e.g. by sonication. For purification of Crf2 in the case of labelled Crf2 containing an added biotin acceptor domain, affinity chromatography was used, followed by dialysis against 20 mM TrisHCl, pH 8 and, optionally, size exclusion chromatography and/or ion exchange chromatography.

### Example 2: Diagnosis of invasive aspergillosis by specific reaction with Crf2

Crf2 gives a highly specific immune reaction with sera from animals that had been exposed to invasive aspergillosis infection, but not with sera from animals suffering from bronchopulmonary aspergillosis or suffering from a *Candida* infection. In the diagnostic method of the invention, the high immune specificity of Crf2 to antibody elicited in animals in response to invasive aspergillosis, especially by *Aspergillus fumigatus* is utilized, which immune reaction does not occur with sera showing immune reactions specific for *Candida* infections or bronchopulmonary aspergillosis.

As an example for an ELISA for detecting the immune reactions of Crf2 with serum, recombinant purified Crf2, optionally containing a label, e.g. a biotin acceptor domain, was immobilized onto a substrate, e.g. onto a polystyrene microtiter plate. After blocking free binding sites with unspecific protein, the serum to be tested was added and incubated to allow for binding of antibody to immobilized Crf2. Following extensive washes to remove nonspecific or unbound antibody, bound antibody was detected, e.g. by a secondary antibody directed against the constant chain of the tested serum, which secondary antibody was coupled to a reporter dye or reporter enzyme.

Preferably, Crf2 without modification, namely consisting of the amino acid sequence of Seq ID No. 1 was immobilized to a carrier substrate followed by blocking and subsequent incubation with the serum to be tested, unbound antibody was removed. Specific interaction of serum antibody with bound Crf2 was by detection using incubation with labelled Crf2, with subsequent washing and specific detection of the label. For detection of the label, a suitable affinity detection or an indicator reaction using the generation of a detectable signal from added specific substrate was used. In this example, labelled Crf2 was Crf2 with an additional N-terminal biotin acceptor domain in combination with detection using a biotinylated reporter. Alternatively, labelled Crf2 was biotinylated Crf2, used in combination with subsequent detection of the biotin label, e.g. by incubation with streptavidin linked to a reporter dye or reporter enzyme, e.g. horse radish peroxidase followed by addition of a reporter substrate convertible by the reporter enzyme.

The diagnostic immunoassay using a first immune reaction of the serum to be tested with immobilized Crf2 with subsequent detection of bound antibody by reaction with labelled Crf2 had the advantage of high specificity while also detecting a large variety of immunoglobulins, including IgG, IgE and IgM.

When coating about 100 ng Crf2 produced according to Example 1 onto microtiter plates (Nunc Maxisorb, Nunc, Wiesbaden), followed by blocking with phosphate buffered saline including 0.1% (vol/vol) Tween20 and 2% (wt/vol) skim milk powder for 1.5 h, Crf2 was immobilized. Washing was with PBS, 0.1% Tween20 without skim milk. Serum samples from experimentally infected mice or patients diagnosed with invasive aspergillosis were diluted about 1: 400 in PBS, Tween20, containing skim milk powder for 1 hour at room temperature. After washing, 100 ng biotinylated Crf2 was added to each well in PBS, Tween20 and incubated for 1 hour. After washing, detection was by incubation with streptavidin-conjugated to horse radish peroxidase, followed by washing and incubation with a substrate for horse radish peroxidase giving an optically detectable signal, e.g. using tetramethyl benzidine as the substrate.

As a result, it was found that only sera from mice or humans diagnosed with invasive aspergillosis yielded a signal in the immuno assay, whereas sera from humans or mice that were diagnosed with bronchopulmonary aspergillosis or a *Candida* infection but without invasive aspergillosis, did not give a positive result in the immunoassay using Crf2.

### Example 3: Crf2 as a vaccine against invasive aspergillosis

The effective immunization against invasive aspergillosis using a vaccine containing Crf2 according to Seq ID No. 1 as the immunogen resulted in an efficient immunoprotection of mice against subsequent infection with conidia of *Aspergillus fumigatus*, which in mock-immunized mice regularly resulted in invasive aspergillosis infections.

C57 BL/6 (H-2b) mice were obtained from Harlan (Germany) and housed under pathogen free conditions. All experiments were approved by the animal protection committee of Braunschweig.

Mice were vaccinated once, followed by a second booster vaccination after 2 weeks, each subcutaneously into a neckfold using 50 µg Crf2 in an aqueous solution in 1:1 (vol/vol) admixture with an adjuvant (TiterMax TM, TiterMax Inc., USA) in a total volume of 60 µL. In the mock immunization, the solution of Crf2 was replaced by an equal volume of sterile PBS.

For the immuno assays, serum was obtained 1 hour before the first immunization, 1 hour before the artificial infection and directly before sacrificing infected mice. Blood was taken from the retro-orbital sinus and serum was separated by centrifugation and optionally frozen at -20 °C before testing using the ELISA.

In order to simulate the impaired function of the immune system in patients suffering from invasive aspergillosis, the experimental animals received 400 µg of RB6-8C5 antibody, which binds to the neutrophil specific antigen Gr-1 (Ly-6G (5)) for deleting neutrophils. Following this immunosuppression, immunized mice were put under a light anesthesia and were inoculated intranasally with 30 µL of a conidial suspension containing 5x10⁶ viable conidia while being held vertically. During recovery from anesthesia, mice were placed on their backs.

1-2 hours following anesthesia and inoculation, all animals recovered and had a normal appearance until first signs of invasive aspergillosis appeared 24-48 hours following infection. For avoiding unnecessary suffering, e.g. laboured breathing, a more than 20% weight loss and occurring specific motile disorders like a heavy rotation along the head - tail - axis, infected animals were killed. Prematurely killed animals were counted as having died from invasive aspergillosis, as was confirmed by later analysis.

Figure 2 A) schematically shows the course of the immunization, consisting of a first immunization with purified Crf2 as the antigen at day -33, followed by a second booster immunization with purified Crf2 at day -19. One day before infection, immunosuppression was caused by intraperitoneal injection of the anti-neutrophil antibody. At day 0, artificial infection of the immunosuppressed animals was performed and mice were observed for the following 7 days.

In Figure 2 B), the number of surviving animals is shown, demonstrating that all the animals immunized with Crf2 survived subsequent to neutrophil depletion and nasal infection with *Aspergillus fumigatus* viable spores, proving that Crf2 is a potent immunogen for a vaccine for providing immunoprotection that is sufficient for protection against later infection by *Aspergillus* even under the conditions of immunosuppression following the immunization with Crf2.

Figure 2 C) shows a Western blot, wherein an SDS-PAGE was loaded with marker protein (left hand lane), 6 lanes loaded with an *E. coli* extract from recombinant *E. coli* expressing Crf2 of Seq ID No. 1, and affinity - purified labelled Crf2, carrying an N-terminal poly-histidine tag (right hand lane).

For detection, serum of one mock-immunized mouse (PBS) was used on separate strips of the Western blot membrane, namely serum taken at day -33, serum taken at day 0 and serum taken at day 7 following artificial infection. All sera were diluted 1:150. The His-tag labelled Crf2 serving as the positive control (pos. ctr1) was separately detected using an anti-His-tag antibody. The reaction of the serum from the mock-immunized mouse at day +7 indicates that this animal had also developed antibody specifically directed against Crf2.

The analysis of the serum from the later Crf2-immunized mouse shows that at day -37, that is prior to immunization, no antibody specific for Crf2 is detected, whereas at day 0, e.g. following the first immunization and the booster immunization with Crf2, a strong antibody reaction has developed, which is detectable through day 7 after the artificial infection with *Aspergillus* conidia.

The immune response at day -33, day 0 and day 7 for the Crf2-immunized mouse serum and the mock-immunized mouse serum used for Figure 2 C) was analysed in the preferred in ELISA using immobilized Crf2, contacting with serum, followed by detection using labelled Crf2, optionally followed by an indicator reaction caused by the label. The results are shown in the Whisker plot of Figure 2 D), showing the negligible background reaction to Crf2 at day -33 for both the later Crf2-immunized and the mock-immunized (PBS) mouse sera. Following the two - staged immunization using Crf2, the Crf2-immunized mouse shows a drastically augmented Crf2-specific immune response, whereas the mock-immunized mouse only shows the original background reaction against Crf2. Following challenge with viable *Aspergillus fumigatus* viable spores, the Crf2-immunized mouse even shows a further increase in the immune response to Crf2, whereas the mock-immunized mouse only at this stage shows an induced antibody reaction.

Figure 3 shows the reaction of sera obtained from the experimental animals in row 1 at day - 33 (d-33), just prior to vaccination with Crf2, at day 0 (d0), i.e. 33 days after vaccination with Crf2, in rows 2 and 3. At day 0, animals were infected with *Aspergillus* conidia and, accordingly, day +7 (d+7) shows the reaction of sera following artificial infection. Generally, in Figure 3, the left column of pictures *Aspergillus fumigatus* and *Candida*, respectively shows immune fluorescence micrographs of sera incubated with *Aspergillus fumigatus* and *Candida* cultivated in RPMI 1640-FCS medium (obtained from PAA, Cölbe, Germany) for 24 h on microscope slides. After cultivation, medium was removed, slides were blocked with blocking buffer (M-PBS, Tween20, 3% BSA) for 1.5 h, then washed with PBS. Following incubation with diluted sera and washing, detection of bound antibody was with goat antimouse (Fc specific), conjugated with Alexa 488 (Invitrogen, Germany), washing with water and observed under a Zeiss Axiovert 200 (Zeiss Jena, Germany) fluorescence microscope. The right column of pictures shows the respective light micrographs.

It can be seen that neither cultivated *Aspergillus fumigatus* hyphae nor *Candida* are recognized by sera prior to Crf2 immunization. Following Crf2 immunization, sera at day 0 recognize *Aspergillus* hyphae but not *Candida*. From the results of the immuno assays, it is concluded that in hyphae, Crf2 protein is detected by sera. When comparing the fluorescence with the light micrograph, it can be seen that sera predominantly react with the hyphae but not with the spore (indicated by arrow), indicating that immunization with Crf2 elicits a protective immune response against actively growing *Aspergillus* cells.

Further, sera taken subsequent to artificial infection (d+7) detect *Aspergillus* hyphae but again not *Candida*. Sera from the mock (PBS) immunized mouse that survived artificial infection only at day 7 (d+7) reacts with *Aspergillus* hyphae, indicating that Crf2 also in infection without prior immunization is the target of the immune response, making Crf2 a suitable vaccine against invasive aspergillosis.

As well as sera from Crf2 immunized animals, sera from the mock immunized mouse neither before nor subsequent to artificial *Aspergillus* infection reacts with *Candida*, proving that Crf2 and the antibodies specific for Crf2 are suitable reactants for specific diagnosis of invasive aspergillosis with differentiation from *Candida* infection, i.e. without reacting with *Candida*.

## Claims

1. Process for a diagnostic assay for detection of the presence of invasive aspergillosis in a animal from an analytical sample obtained from the animal, **characterized by** comprising the steps of
contacting a protein comprising the amino acid sequence of Crf2 of Seq ID No. 1 with the analytical sample, and
identifying binding of antibody of the analytical sample to the protein comprising the amino acid sequence of Crf2.

2. Process according to claim 1, **characterized** that subsequent to contacting the analytical sample with the protein comprising the amino acid sequence of Crf2, the analytical sample is contacted with a labelled protein comprising the amino acid sequence of Seq ID No. 1, followed by detection of the label of the labelled protein.

3. Process according to claim 1 or 2, **characterized** that the protein comprising Seq ID No. 1 consists of Crf2 according to Seq ID No. 1.

4. Process according to one of the preceding claims, **characterized in that** the labelled protein is labelled Crf2 consisting of amino acid sequence Seq ID No. 1.

5. Process according to one of the preceding claims, **characterized in that** the label is selected from a poly-histidine sequence, a biotin acceptor domain, biotinylation, a strep-tag, flag-tag, E-tag, S-tag, myc-tag and yol-tag.

6. Process according to one of the preceding claims, **characterized by** its use for differentiation of invasive aspergillosis from bronchopulmonary aspergillosis and/or from an infection by *Candida*.

7. Test kit for diagnosing invasive aspergillosis in a patient according to a process of one of the preceding claims, **characterized by** comprising a protein comprising the amino acid sequence of Crf2 of Seq ID No. 1.

8. Test kit according to claim 7, **characterized in that** Crf2 in addition to amino acid sequence Seq ID No. 1 contains at least one amino acid sequence section as a label.

9. Test kit according to one of claims 7 or 8, **characterized in that** the label is selected from biotinylation, a poly-histidine sequence, a biotin acceptor domain, strep-tag, flag-tag, E-tag, S-tag, myc-tag and yol-tag.

10. Use of Crf2 comprising an amino acid sequence of Seq ID No. 1 in a test kit according to one of claims 7 to 9.

11. Pharmaceutical composition containing an immunogen for eliciting an immune response directed against aspergillosis in a animal, **characterized in that** the immunogen is Crf2 consisting of the amino acid sequence of Seq ID No. 1.

12. Pharmaceutical composition according to claim 11, **characterized in that** the immunogen has the same immunogenic properties as the amino acid sequence of Seq ID No. 1, contains at least one epitope of Seq ID No. 1 and differs from Seq ID No. 1.

13. Pharmaceutical composition according to claim 11 or 12, **characterized in that** at least following administration of the pharmaceutical composition the animal has an impaired immune system.

14. Pharmaceutical composition according to claim 13, **characterized in that** the impairment of the immune system is caused by an HIV infection or by the administration of immunosuppressive compounds.

15. Pharmaceutical composition according to one of claims 13 or 14, **characterized in that** the impairment of the immune system is the depletion of neutrophils.

16. Pharmaceutical composition according to one of claims 11 to 15, **characterized in that** the animal is a human or a bird.

17. Use of a protein comprising Seq. ID No. 1 for the production of a pharmaceutical composition for eliciting an immune response in an animal, **characterized in that** the protein consists of Crf2 having Seq. ID No. 1 and the immune response is protective against invasive aspergillosis.

18. Use according to claim 17, **characterized in that** the animal has an impaired immune system including the depletion of neutrophils.
